# EUROPEAN PATENT SPECIFICATION

(11) **EP 1 078 931 B1**
(45) Date of publication and mention of the grant of the patent: **08.03.2006**
(21) Application number: 00117261.8
(22) Date of filing: 16.08.2000
(51) Int. Cl.: C07F 7/18, C07D 303/40, C07D 303/12

(54) **Process for the preparation of vitamin D analogues**
Verfahren zur Herstellung von Vitamin-D Analogen
Procédé de préparation d'analogues de vitamine D

(30) Priority: 23.08.1999 US 150226 P; 23.08.1999 US 150378 P
(43) Date of publication of application: 28.02.2001
(73) Proprietor: F. HOFFMANN-LA ROCHE AG, 4070 Basel (CH)
(72) Inventor: Daniewski, Andrzej Robert, Bloomfield, New Jersey 07003 (US); Kabat, Marek Michal, Nutley, New Jersey 07110 (US); Okabe, Masami, Nutley, New Jersey 07110 (US); Radinov, Rouman Nikolaev, Caldwell, New Jersey 07006 (US)
(74) Representative: Kjellsaa-Berger, Hanny

(56) References cited:
- EP-A- 0 503 630
- SHIUEY, S.-J.: "Total Synthesis of 1-alpha-Fluoro-25-hydroxycholecalciferol and -ergocalciferol" J.ORG.CHEM., no. 55, 1990, pages 243-247, XP002216706
- DATABASE CROSSFIRE BEILSTEIN [Online] Beilstein Institut zur Förderung der Chemischen Wissenschaften, Frankfurt am Main, DE; Database accession no. BRN 4185651 XP002229621 & KANG, JAHO: J.ORG.CHEM, no. 55, 1990, pages 5555-5558,

## Description

The invention relates to a process useful to produce vitamin D analogs, such as calcitriol, sold under the brand name Rocaltrol®.

Processes for manufacturing vitamin D analogs typically require multiple steps and chromatographic purification. See, Norman, A. W.; Okamura, W. H. PCT Int. Appl. WO 9916452 A1 990408; *Chem Abstr.* 130:282223. Batcho, A. D.; Bryce, G. F.; Hennessy, B. M.; Iacobelli, J. A.; Uskokovic, M. R. Eur. Pat. Appl. EP 808833,1997; *Chem. Abstr.* 128:48406. Nestor, J. J.; Manchand, P. S.; Uskokovic, M. R. Vickery, B. H. US 5872113, 1997; *Chem. Abstr.* 130:168545. The present invention seeks to provide an efficient synthesis of the A-ring portion of such vitamin D analogs.

The subject invention provides a method of stereospecifically producing a compound of formula: 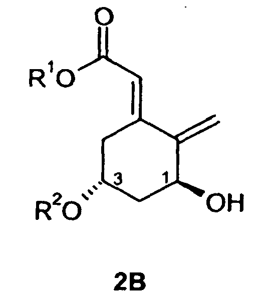
or the enantiomer thereof 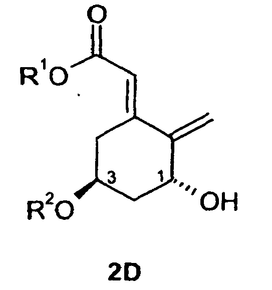
wherein R¹ is C₁-C₆ alkyl and R² is a hydroxy protecting group,
which comprises
reacting a compound of formula: 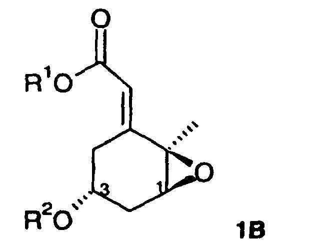
or its enantiomer, respectively, 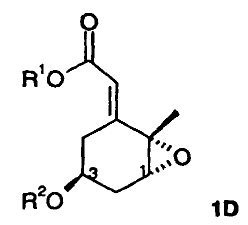
wherein R¹ and R² are as above,
and the stereochemistry of the compound of formula 1B, and the compound of formula 2B is the same at carbons 1 and 3, respectively, and the stereochemistry of both the compound of formula 1D and the compound of formula 2D is the same at carbons 1 and 3, respectively,
with a fluorinated alcohol having a pKₐ lower than about 9, in the presence of a palladium catalyst to yield the compound of formula 2B or 2D respectively.

The reacting is preferably in the presence of a palladium catalyst that is palladium-phosphine catalyst, such as a palladium-triarylphosphine, especially when selected from the group consisting of palladium-triphenylphosphine, palladium-tris(2-methoxyphenyl)-phosphine, palladium-tris(3-methoxyphenyl)phosphine, palladium-tris(4-methoxyphenyl)phosphine, palladium-tris(o-tolyl)phosphine, palladium-tris(m-tolyl)phosphine, palladium-tris(p-tolyl)phosphine, palladium-tris(4-fluorophenyl)phosphine, palladium-tris(p-trifluoromethylphenyl)phosphine, and palladium-tris(2-furyl)phosphine. Another preferred palladium catalyst is palladium-1,2-bis(diphenylphosphino) ethane.

The fluorinated alcohol is favorably selected from the group consisting of: 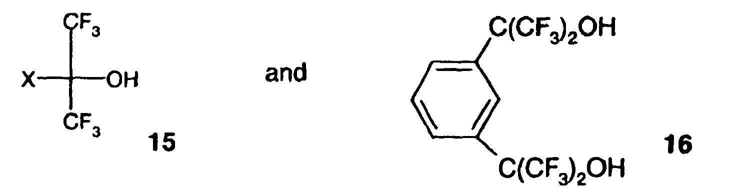
wherein X is phenyl or CF₃;
for example: 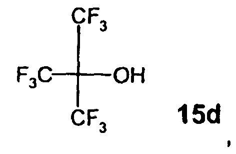 or 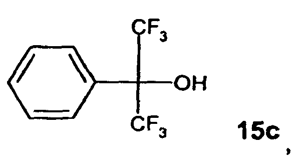 or

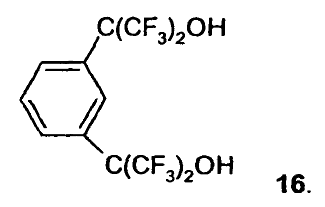

The present invention also provides novel intermediates used in the process for the preparation of the compounds of formulae 2B and 2D, the invention is thus related to novel intermediate having the formula 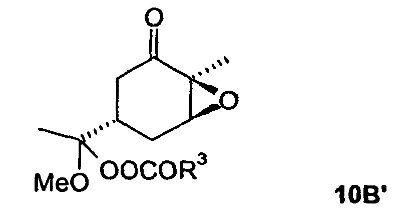
wherein R³ is C₁-C₆ alkyl, phenyl, 4-nitrophenyl, or CF₃; preferably to the intermediates of formulae

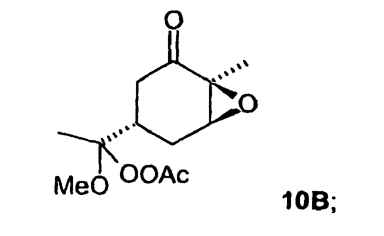

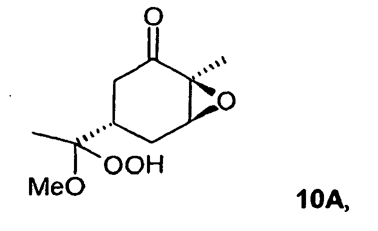

Further the invention relates to the compound having the structure: 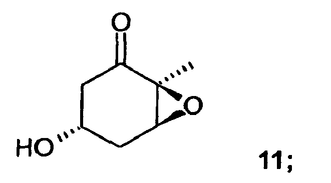
and to the compounds having the structure: 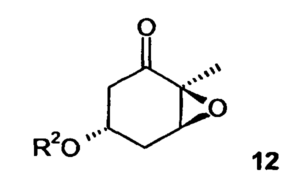
wherein
- R²: is a hydroxy protective group selected from the group consisting of trimethylsilyl, triethylsilyl, tripropylsilyl, triisopropylsilyl, *t*-butyldimethylsilyl, dimethylthexylsilyl, triphenylsilyl, and *t*-butyldiphenylsilyl;

and preferably to the compound having the structure:

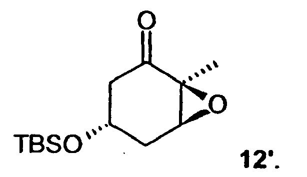

Further novel compounds include the compound having the structure: 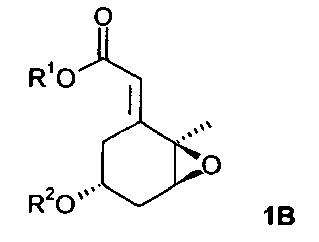
wherein
- R¹: is C₁-C₆ alkyl and R² is a hydroxy protective group selected from the group consisting of trimethylsilyl, triethylsilyl, tripropylsilyl, triisopropylsilyl, *t*-butyldimethylsilyl, dimethylthexylsilyl, triphenylsilyl, and *t*-butyldiphenylsilyl;

examples of such compounds are the compounds having the structure: 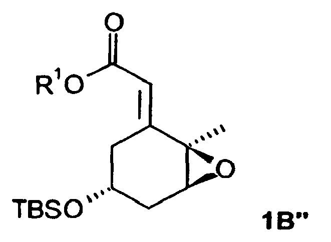
wherein R¹ is C₁-C₆ alkyl;
or the compounds having the structure: 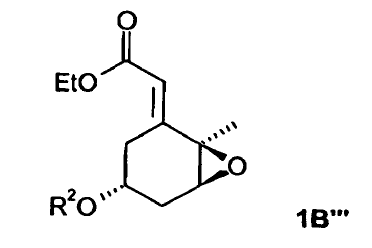
wherein
- R²: is a hydroxy protective group selected from the group consisting of trimethylsilyl, triethylsilyl, tripropylsilyl, triisopropylsilyl, *t*-butyldimethylsilyl, dimethylthexylsilyl, triphenylsilyl, and *t*-butyldiphenylsilyl;

preferably the compound having the structure:

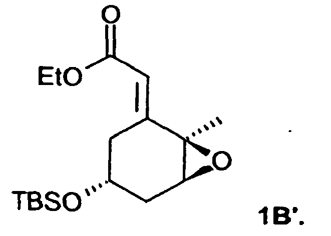

The enantiomeres of the novel intermediates and compounds mentioned above are also part of the present invention.

The subject invention will now be described in terms of its preferred embodiments. These embodiments are set forth to aid in understanding the invention but are not to be construed as limiting.

The subject invention is concerned generally with a stereospecific and regioselective process for converting compounds of formula 1 to compounds of formula 2. However, as explained below, there are certain differences between the processes involving compounds of formula 1 wherein the substituents at the 1 and 3 carbons are attached *cis-,* i.e. on the same side of the plane of the six-membered ring, and compounds of formula 1 wherein the substituents at the 1 and 3 carbons are attached *trans-,* i.e. on opposite sides of the plane of the six-membered ring.

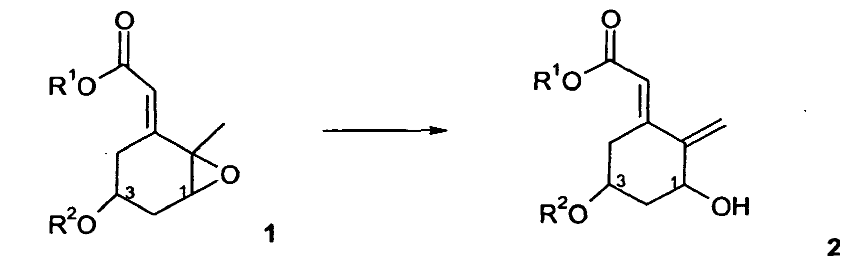

The process results in the compound of formula 2 having the same relative and absolute stereochemistry at both carbon 1 and carbon 3 as that in the compound of formula 1. Thus, if carbon 1 is in the R-configuration in the compound of formula 1, then carbon 1 will be in the R-configuration in the compound of resulting formula 2. In the above process, R¹ is C₁-C₆ alkyl that can be straight-chain or branched. For example, methyl, ethyl, propyl, isopropyl, butyl (primary, secondary or tertiary), pentyl (primary, secondary or tertiary), or hexyl (primary, secondary or tertiary). R² is a hydroxy protective group. The choice of protective group is readily determinable by the skilled artisan. However, a silyl protective group, such as *tert*-butyldimethylsilyl ("TBS") is preferred.

The bonds forming the epoxide ring may be above the plane or below the plane of the molecule. When the epoxide ring is below the plane, the adjacent methyl group is above the plane. Likewise, when the epoxide ring is above the plane, the adjacent methyl is below the plane.

When the substituents at carbons 1 and 3 are *trans,* the following situations can occur:

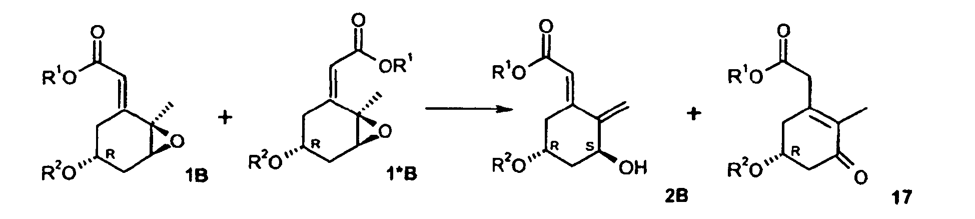

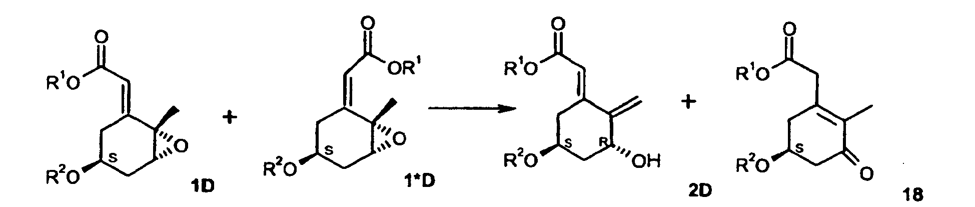

Compounds of formula 2B-D are useful for the preparation of Vitamin D analogs, for example, for compound 2B, see: Nagasawa, K.; Zako, Y; Ishihara, H.; Shimizu, I. *Tetrahedron Lett.* 1991, 32, 4937. Nagasawa, K.; Zako, Y.; Ishihara, H.; Shimizu, I. *J*. *Org. Chem.* 1993, 58, 2523; for compound 2D, see: Shimizu, N. Jpn. Kokai Tokkyo Koho JP 04305553 A2 921028; *Chem. Abstr.* 118:191249. Shimizu, N. Jpn. Kokai Tokkyo Koho JP 04305548 A2 921028; *Chem. Abstr.* 118:212477. Minojima; T.; Tomimori, K.; Kato, Y. Jpn. Kokai Tokkyo Koho JP 02286647 A2 901126; *Chem. Abstr.* 114:184872.

The choice of hydroxy protective group is readily apparent to the skilled artisan, see for example T. W. Greene, P. G. M. Wuts, Protective Groups in Organic Synthesis, 2^{nd} Ed., John Wiley & Sons, 1991. Acceptable hydroxy protective groups for use in connection with the subject invention include silyl ethers such as trimethylsilyl, triethylsilyl, tripropylsilyl, triisopropylsilyl, t-butyldimethylsilyl, dimethylthexylsilyl, triphenylsilyl, and *t*-butyldiphenylsilyl.

Compounds of formula 1B and 1D are enantiomers, and can be prepared from known compounds. For example, the starting material may be (+)-Carvone [Okamura, W H.; Aurrecoechea, J. M.; Gibbs, R. A.; Norman, A. W *J. Org. Chem.* 1989, *54,* 4072] for the preparation of 1B, and the starting material may be (-)-Carvone [Jones, Joel, Jr.; Kover, W B. *Synth. Commun.* 1995, *25*, 3907] for the praparation of 1D. Thus, compound 9 or its enantiomer may be obtained from (+)-Carvone or (-)-Carvone, respectively, by diastereoselective epoxidation according to procedures set forth in the above publications. A skilled chemist having read the present specification would know how to produce a given enantiomer by choosing the corresponding enantiomeric starting material.

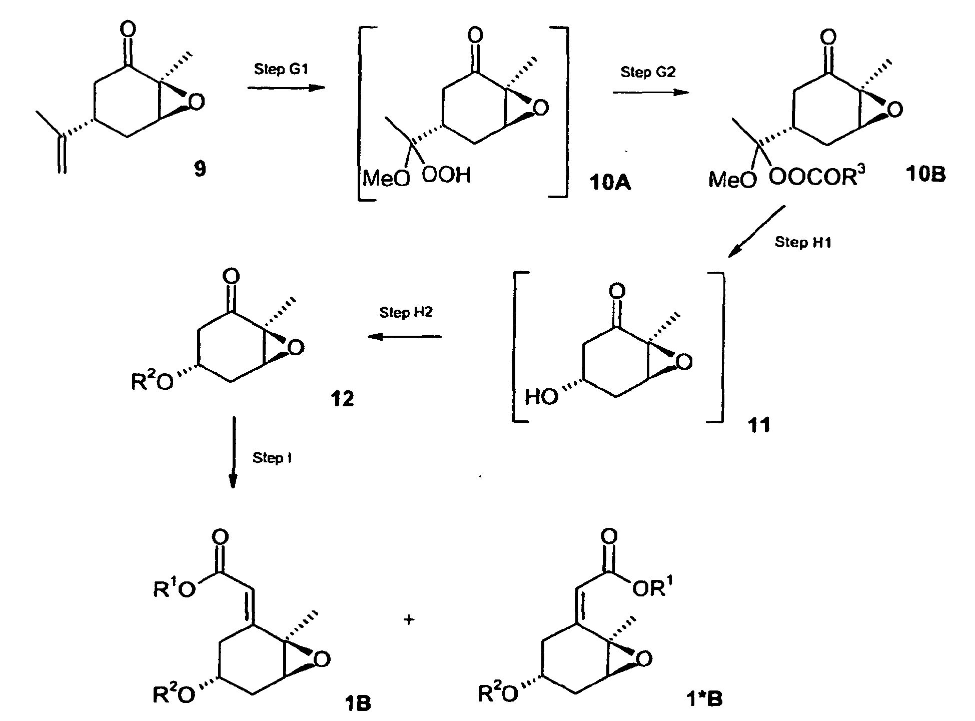

### Step G

The compound of formula 9 is known [Klein, E.; Ohloff, G. *Tetrahedron* 1963,19, 1091. Okamura, W. H.; Aurrecoechea, J. M.; Gibbs, R. A.; Norman, A. W. *J*. *Org. Chem.* 1989,54,4072].

At low temperature (-70°C) a 1,3-dipolar cydoaddition of ozone to the compound of formula 9 occurs to give an ozonide, which at a higher temperature (e.g., room temperature) releases formaldehyde via a retro-1,3-dipolar cycloaddition to form carbonyl oxide. In the presence of methanol as a co-solvent, the carbonyl oxide is efficiently trapped by the alcohol to give the desired hydroperoxide of formula 10A (Step G1) which is then acylated to the compound of formula 10B (Step G2). Variations on common acylation are readily apparent to one of ordinary skill of the art. In the compound of formula 10B, R³ can be C₁-C₆ alkyl, phenyl, 4-nitrophenyl, or CF₃. Such variations are readily made by the skilled artisan.

Excess methanol may interfere with this acylation. However, a dean reaction can be achieved with 4 equivalents of methanol. Then, the hydroperoxide can be acetylated *in situ* with 7 equivalents of acetic anhydride and triethylamine in the presence of a catalytic amount of DMAP at -5°C to obtain peroxyacetate 10B, where R is a methyl group. Other acylating agents may be similarly used and the resulting peroxyester subjected to the Criegee rearrangement as described below. Such apropriate acylating agents are aliphatic and aromatic acid halides (chlorides or bromides) and acid anhydrides, such as acetylchloride, acetic anhydride, propionylchloride, benzoylchloride, 4-nitrobenzoylchloride, and trifluoroacetic anhydride. These acylating agents may react with hydroperoxide 10A in the presence of base such as triethylamine, as above, to give the corresponding peroxyesters 10B, where R is methyl, ethyl, phenyl, 4-nitrophenyl, trifluoromethyl. However, a peroxyacetate 10B where R is methyl, is preferred.

### Step H1

The peroxyester of formula 10B is immediately subjected to the Criegee rearangement to yield the alcohol of formula 11, preferably in methanol. The peroxyacetate of formula 10B tends to be unstable. Accordingly, sodium acetate may be added to prevent acid-catalyzed solvolysis of the compound of formula 10 to the corresponding dimethyl acetal and Step H1 preferably follows Step G immediately. An aqueous workup of the reaction mixture should be used to remove acidic and basic by-products in order to obtain purified compound of formula 11.

### Step H2

After solvent exchange with acetonitrile, the product of formula 11 can be protected (for example, silylated) to give the ketone of formula 12. The relatively volatile protective group (for example, silyl) by-products can be removed at 45°C under high vacuum and the crude product of formula 12 obtained.

Protection of the secondary alcohol in formula 11 can be achieved using known protection technology, for example using t-butyldimethylsilyl chloride and imidazole. Other silyl protective groups, such as trimethylsilyl, triethylsilyl, tripropylsilyl, triisopropylsilyl, dimethylthexylsilyl, triphenylsilyl, and *t*-butyldiphenylsilyl protective groups can be similarly used, when a corresponding silylchloride is reacted with 7 in the presence of base, such as imidazole, pyridine, or other aromatic or aliphatic tertiary amine under controlled conditions to minimize elimination of the silyloxy group.

It is noteworthy that the product of the Criegee rearrangement in methanol is the alcohol of formula 11 and that the corresponding acetate ester has never been observed in the course of the reaction. This contrasts to the typical Criegee rearrangement procedure (one-pot acetylation and rearrangement in dichloromethane: Schreiber, S. L; Liew, W. F. *Tetrahedron Lett.* 1983, *24*, 2363), where an acetate is usually obtained as the major product together with a smaller amount of the corresponding alcohol. Subsequent hydrolysis of the acetate to the alcohol is problematic due to elimination of the acetoxy group.

### Step I

A Wittig-Horner reaction of the compound of formula 12 can be carried out using 2.2 equiv. of tri-R¹ phosphonoacetate (where R¹ is a C₁-C₆ alkyl that can be straight-chain or branched) and 1.8 equiv. of lithium hydride in a relatively small amount of THF, at a relatively low temperature (11°C), for a longer reaction time (20 h) to minimize elimination of the protecting (for example, silyloxy) group. The desired compound of formula 1B is thus obtained in approximately a 7-9:1 mixture with its Z-isomer (the compound of formula 1 *B).

To illustrate the inventive aspects of the subject reaction, the reaction will be discussed with reference to the reaction of a species of formula 1B" to form the corresponding species of formula 2B". The same principles hold true for its enantiomer, compound 1D to form 2D.

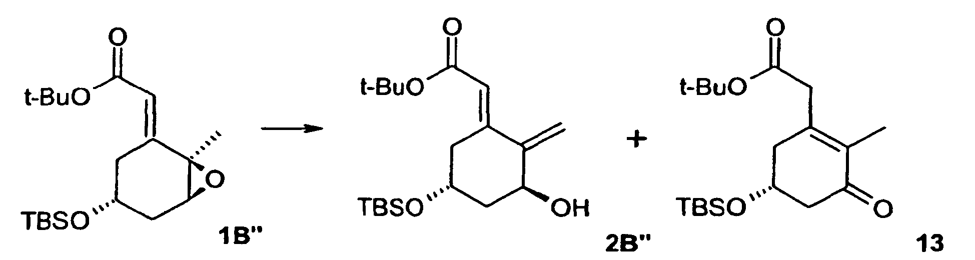

The above reaction, when using a palladium(0) triphenylphosphine catalyst [Suzuki, M.; Oda, Y.; Noyori, R. *J*. *Am. Chem. Soc.* 1979,*101*, 1623J in THF at 65°C, results in the isomerization of epoxide 1B" to yield a mixture of the desired allyl alcohol of formula 2B" and isomeric enone of formula 13 in a ratio of 1:3 (HPLC area% at 220 nm). It has been discovered that phosphine ligands [for example, triarylphosphines, such as triphenylphosphine, tris(2-methoxyphenyl)phosphine, tris(3-methoxyphenyl)phosphine, tris(4-methoxyphenyl)phosphine, tris(o-tolyl)phosphine, tris(m-tolyl)phosphine, tris(p-tolyl)-phosphine, tris(4-fluorophenyl)phosphine, tris(p-trifluoromethylphenyl)phosphine, and tris(2-furyl)phosphine, and aryl phosphines such as 1,2-bis(diphenylphosphino)ethane] in combination with palladium(0) catalyze the isomerization and that adding a fluorinated alcohol [for example 1,1,1,3,3,3-hexafluoro-2-phenyl-2-propanol and 1,3-bis(1,1,1,3,3,3-hexafluoro-2-hydroxypropyl)benzene, perfluoro-*t*-butanol] increases the yield of the desired allyl alcohol of formula 2B" *versus* the undesired ketone of formula 13 and also improves catalyst turnover for the palladium-triphenylphosphine catalyst. The palladium-phosphine catalyst can be prepared *in situ* prior to the reaction from commercial palladium sources, such as Pd₂dba₃(CHCl₃) ("dba" stands for dibenzylideneacetone), and an excess (typically 4-5 equivalents) of the corresponding phosphine ligand, such as triphenylphosphine. Other palladium sources may be used as well, such as palladium(0) complexes Pd₂dba₃, Pddba₂, and palladium(II) salts Pd(OAc)₂, PdCl₂, [allylPdCl]₂, and Pd(acac)₂ ("acac" stands for acetylacetonate). Alternatively, a palladium(0)-phosphine catalyst, such as tetrakis(triphenylphosphine)palladium(0), may be separately prepared and used in the reaction. However, generation of the catalyst *in situ* from Pd₂dba₃(CHCl₃) and phosphine is preferred. With 1 mol% of the palladium-triphenylphosphine catalyst even a catalytic amount of the appropriate fluorinated alcohol was sufficient to increase the selectivity for allyl alcohol of formula 2B" to 10:1. Increasing the amount of fluorinated alcohol of formula 15c further to 50 mol% and 100 mol% gave a 16:1 and 19:1 ratio of allyl alcohol of formula 2B" to isomeric enone of formula 13, respectively. 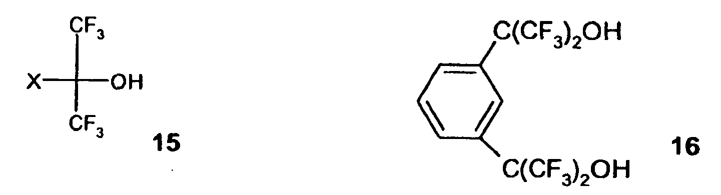
where X is CH₃ (formula 15a), H (formula 15b), phenyl (formula 15c), or CF₃ (formula 15d).

It has been discovered that selectivity correlated to the pKₐ of the fluorinated alcohols. Fluorinated alcohols with pKₐ <9 were particularly effective.

A 7:1 mixture of the compound of formula 1B" (formula 1B" is the formula 1B wherein R¹ is t-Bu and R² is TBS) and the compound of formula 1*B" (the Z-isomer of the compound of formula 1B") was subjected to the palladium catalyzed isomerization reaction as described above to yield a 88:12 mixture of the desired allylic alcohol of formula 2B" (formula 2B" is the formula 2B wherein R¹ is t-Bu and R² is TBS) and its corresponding ketone (see the following Table). Thus, the regioselectivity depends on the stereochemistry of the diene oxide double bond. Isomers 1B (E-isomer) and 1*B (Z-isomer) can be separated by chromatography. From pure E-isomers 1B the desired allylic alcohols (2B" and 2B") were obtained with high selectivity (>99%). On the other hand, (Z)-diene oxides 1*B gave ketones 13 and 14 selectively (see the table below). Both ethyl and t-butyl esters gave similar results.

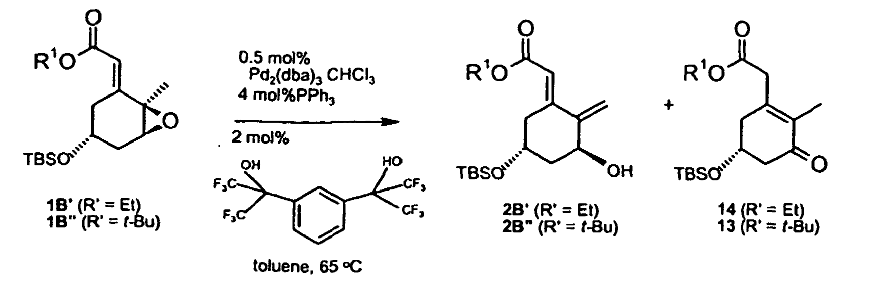

| R¹ (Substrate) | E: Z of substrate | E : Z of product | Allylic Alcohol : Ketone |
|---|---|---|---|
| Et (1B' + 1*B') | 4 : 1 | E only (2B') | 80:20 (2B':14) |
| Et (1B' + 1*B') | 7 : 1 | E only (2B') | 88:12 (2B':14) |
| Et (1B' + 1*B') | 9 : 1 | E only (2B') | 90 : 10 (2B':14) |
| Et (1B') | E only | E only (2B') | >99 : 1 (2B':14) |
| Et (1*B') | Z only | E only (2B') | 14 : 86 (2B':14) |
| *t*-Bu (1B") | E only | E only (2B") | >99 : 1 (2B":13) |
| *t*-Bu (1*B") | Z only | E only (2B") | 8 : 92 (2B":13) |

Although a high selectivity (>99%) was achieved with the pure E-isomers of formula 1B, under commercial conditions it may not be practical to separate the E-isomers 1B from the Z-isomers 1*B. Thus, in practice a mixture of E/Z-isomers will typically be subjected to the epoxide opening and, after solvent exchange with DMF, the resulting mixture of allylic alcohol 2B'/2B" and ketone 14/13 will be subjected to silylation. Silylation is typically achieved using t-butyldimethylsilyl chloride and imidazole using known protection technology. Other silyl protective groups, such as trimethylsilyl, triethylsilyl, tripropylsilyl, triisopropylsilyl, dimethylthexylsilyl, triphenylsilyl, and *t*-butyldiphenylsilyl protective groups can be similarly used, when a corresponding silylchloride is reacted with alcohol 2B. Since alcohol 2B'/2B" is converted to a non-polar product by silylation, while the polar ketone remains unchanged, pure silylated product can be easily isolated by a simple silica gel filtration.

The following examples were actually performed and are illustrative of the invention. Modifications of these examples to produce related compounds as shown in the various schemes herein are obvious chemical modifications to a person of ordinary skill in the art.

### Example 1 - Preparation of Diene-Ester of Formula 3B'

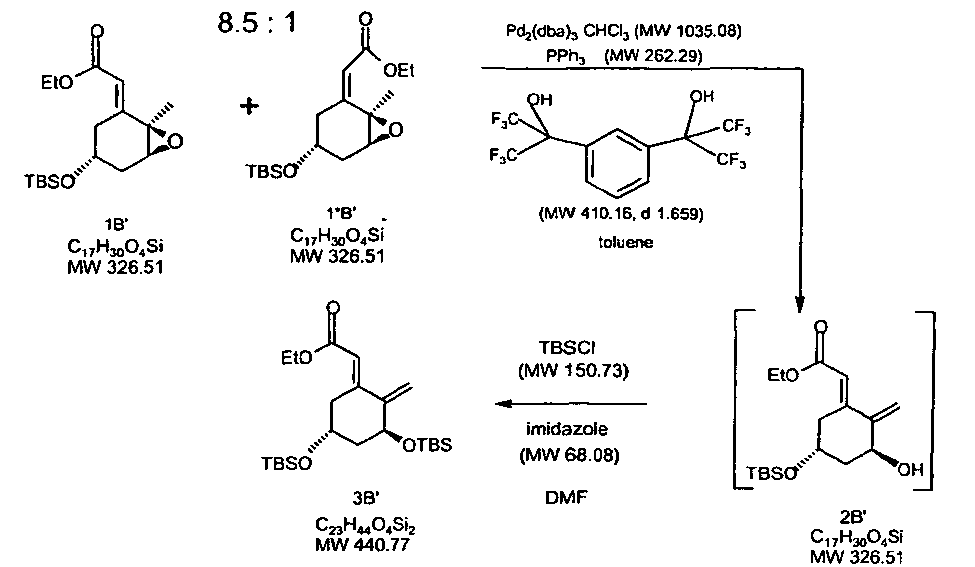

A 250 mL round-bottomed flask equipped with a magnetic stirrer, septum stopper, thermocouple and nitrogen bubbler was charged with 388 mg (0.375 mmol) of tris(dibenzylideneacetone)dipalladium(0)-chloroform adduct and 985 mg (3.75 mmol) of triphenylphosphine. The flask was evacuated and refilled with nitrogen three times, then charged via syringe with 23 mL of toluene. The resulting deep purple mixture was stirred at ambient temperature for 30 min to give a light orange suspension. Then, 370 µL (1.5 mmol) of 1,3-bis-(1,1,1,3,3,3-hexafluoro-2-hydroxypropyl)benzene was added. The mixture turned red-orange and most of the solids dissolved. After three minutes of stirring at ambient temperature (19°C), to the resulting catalyst solution was added, via cannula using a slightly positive nitrogen pressure, a solution of 24.4 g (74.9 mmol) of crude compound of formula 1B'/1*B' (E/Z 8.5:1) in 100 mL of toluene, prepared in a similar manner to that described above for the catalyst solution (the flask containing the crude compound of formula 1B' was evacuated and refilled with nitrogen three times, then the toluene was added via cannula). After ten minutes of stirring at ambient temperature under slightly positive nitrogen pressure, the reaction mixture was heated to 40°C overnight (16 hours). TLC analysis indicated complete reaction. The mixture was concentrated on a rotary evaporator at <40°C under reduced pressure to remove most of the toluene. The resulting brown oil was dissolved in 80 mL of DMF and the resulting solution was cooled with an ice-water bath, then 6.12 g (89.8 mmol) of imidazole followed by 13.5 g (89.8 mmol) of *t*-butylchlorodimethylsilane were added. After 10 min, the cooling bath was removed and stirring was continued at room temperature overnight. TLC analysis indicated complete reaction. The reaction mixture was diluted with 300 mL of hexanes and washed with 2x150 mL = 300 mL of water. The combined aqueous washes were back-extracted with 2x100 mL = 200 mL of hexanes and the combined back-extracts were washed with 2x50 mL = 100 mL of water. All the organic layers were combined, dried over magnesium sulfate and concentrated to dryness to give a yellow, viscous oil (35.6 g, overweight). This material was dissolved in 100 mL of hexanes and the resulting solution was filtered through 200 g of TLC silica gel. The silica gel pad was then washed with 1.5 L of 98:2 hexane:ethyl acetate, and the combined filtrate and washes were concentrated to dryness under reduced pressure to give 27.7 g (84.0%) of the compound of formula 3B' as a colorless oil.

In-process controls: HPLC, NMR (CDCl₃) and TLC (3:1 pet.ether:diethyl ether; short-wave UV detection and PMA stain; R_{f}3B' = 0.9, R_{f}1B' = 0.85, R_{f}2B' = 0.45, R_{f}14 = 0.6 and R_{f} of dibenzylideneacetone = 0.7, 19:1 hexane:ethyl acetate; short-wave UV detection and PMA stain; R_{f} of the compound of formula 3B' = 0.4 and R_{f} of the compound of formula 2B' =0.1)

### Preparatory Example 1 - Preparation of the Peroxyacetate of formula 10

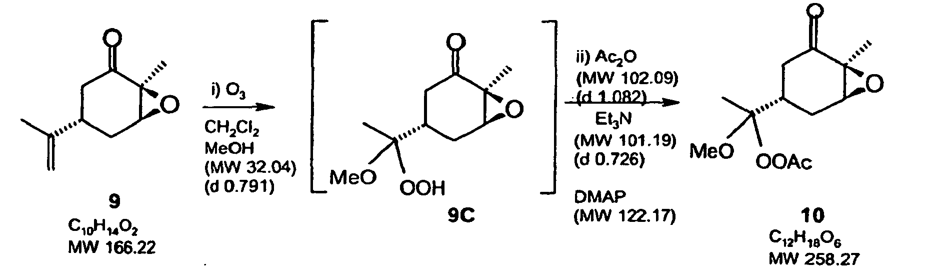

A 500 mL, three-necked, round-bottomed flask equipped with a mechanical stirrer, thermometer, nitrogen inlet-tube and gas outlet-tube was charged with 20.0 g (120 mmol) of the compound of formula 9, 20 mL (494 mmol) of methanol and 200 mL of dichloromethane. After the mixture was cooled to -68°C with a dry-ice/acetone bath, the nitrogen inlet-tube was replaced with a gas dispersion tube with porous fritted glass tip (25-50µ), and the gas outlet-tube was connected, through a trap, to a tube (4 mm I.D.) immersed in a 1M solution of potassium iodide (2 L). Then, ozonized air (4.5 LPM) was continuously passed through the reaction mixture at -68 ± 3°C. The reaction turned pale blue after 65 min, indicating complete reaction. Excess ozone was removed by purging with nitrogen for 30 min to give a colorless solution. The gas dispersion and outlet tubes were replaced with a nitrogen bubbler and an addition funnel. The mixture was allowed to warm to 14°C over 40 min. After cooling to -25°C with a dry-ice/acetone bath, 117 mL (839 mmol) of triethylamine was added over 5 min, while maintaining the temperature of the mixture below -25°C. Then, 2.0 g (16.4 mmol) of dimethylaminopyridine. (DMAP) was added in one portion and 79.6 mL (843 mmol) of acetic anhydride was added slowly over 10 min, while maintaining the reaction temperature between -25°C and -38°C. The mixture was allowed to warm to -8°C over 30 min and stirred at -7 ± 1°C for 1.5 h. TLC analysis indicated complete reaction. The reaction mixture was quenched by the slow addition (over 7 min) of 33 mL of methanol, while maintaining the temperature of the mixture below 10°C. After stirring for 5 min at 5°C, the mixture was diluted with 220 mL of hexane, washed with 2x150 mL = 300 mL of 10% citric acid solution and 2x80 mL = 160 mL of saturated potassium bicarbonate solution, dried over sodium sulfate and concentrated to dryness at 35°C under reduced pressure to give 38.2 g (overweight) of crude the compound of formula 10 as a yellow oil. This material was immediately used in the next step without further purification.

In-process controls: NMR (CDCl₃) and TLCs (2:1 hexane:ethyl acetate; PMA stain; R_{f} the compound of formula 9 = 0.80 and R_{f} the compound of formula 9C = 0.45,40:2:1 dichloromethane:ethyl acetate:methanol; PMA stain; R_{f} the compound of formula 9C = 0.40 and R_{f} the compound of formula 10 = 0.80).

### Preparatory Example 2 - Preparation of the Ketone of formula 12'

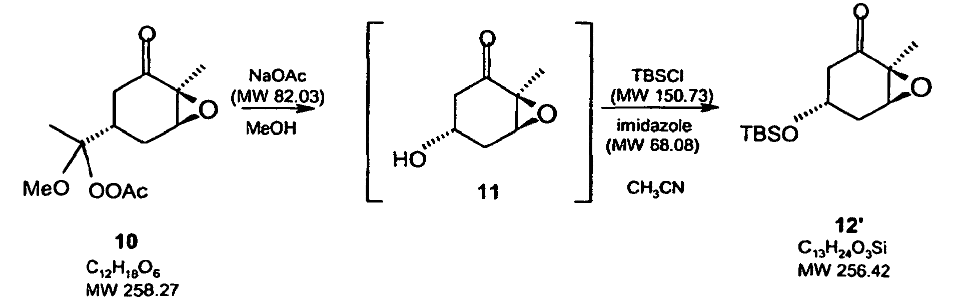

A 500 mL round-bottomed flask equipped with a magnetic stirrer, thermometer and nitrogen bubbler was charged with 38.2 g (120 mmol, theoretical) of crude compound of formula 10. 2 g (24.4 mmol) of sodium acetate and 245 mL of methanol. After stirring at 37°C overnight, TLC analysis indicated complete reaction. Thus, the mixture was concentrated to dryness at 39°C and the residue (29 g) was dissolved in 40 mL of acetonitrile. The resulting solution was concentrated to dryness at 35°C under reduced pressure and 40 mL of acetonitrile was added. The resulting solution was again concentrated to dryness at 35°C under reduced pressure, and 35 mL of acetonitrile and 29.5 g (433 mmol) of imidazole were added. After cooling with an ice-water bath, 32.6 g (217 mmol) of tert-butylchlorodimethylsilane was added. The cold bath was removed and the mixture was stirred at room temperature for 4 h. TLC analysis indicated the presence of only a trace amount of starting material. The reaction mixture was quenched by the addition of 10 mL of methanol. A mild exotherm ensued that raised the temperature of the mixture by 2°C. After stirring for 5 min, 55 mL of ice water was added and the mixture was extracted with 2x50 mL = 100 mL of hexane. The combined organic layers were washed with 50 mL of 2:3 methanol:water, dried over sodium sulfate and concentrated to dryness at 40°C under reduced pressure. Further drying of the residue at 46°C and 0.4 mmHg for 1 h gave 25.2 g of crude compound of formula 12' as a pale yellow oil. This material was used directly in the next step without further purification.

In-process controls: NMR (CDCl₃) and TLCs (40:2:1 dichloro-methane:ethyl acetate:methanol; PMA stain; R_{f} the compound of formula 10 = 0.8, R_{f} the compound of formula 11 = 0.4 and R_{f} compound of formula 12' = 0.95, 8:1 hexane:ethyl acetate; PMA stain; R_{f} the compound of formula 12' = 0.6 and R_{f} of *tert*-butyldimethylsilanol = 0.5)

### Preparatory Example 3 - Preparation of the Unsaturated Ester of formula 1B'

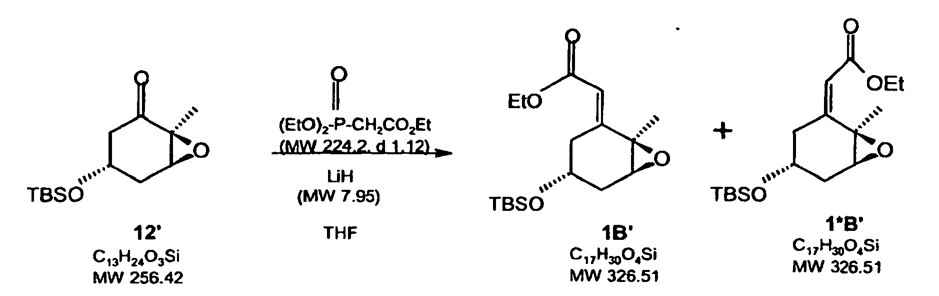

A 250 mL, three-necked, round-bottomed flask equipped with a magnetic stirrer, condenser, thermometer and nitrogen bubbler was charged with 1.41 g (177 mmol) of lithium hydride, 43.3 mL (216 mmol) of triethyl phosphonoacetate and 45 mL of THF. The mixture was slowly heated to 55°C and the heating bath was removed. An exotherm ensued that raised the temperature of the mixture to 69°C over 5 min. The temperature of the mixture slowly came down to 66°C over 55 min and a clear solution resulted. Approximately 25 mL of the THF was then removed by distillation at 50-55°C under a slightly reduced pressure. After cooling the resulting mixture to 3°C with an ice water bath, 25.2 g (98.4 mmol) of crude the compound of formula 12' was added in one portion. The funnel was rinsed with 15 mL of THF and the rinse was added to the reaction mixture. The mixture was stirred at 5-6°C for 90 min, at 11°C for 18 h, then at 24°C for 2 h. TLC analysis indicated complete reaction. Thus, the mixture was diluted with 100 mL of 8:1 hexane:ethyl acetate, washed with 3x36 mL = 108 mL of water and concentrated to dryness at 38°C under reduced pressure. The residue was dissolved in 115 mL of hexane and filtered through 50 g of TLC silica gel. The silica gel pad was then washed with 191 mL of 8:1 hexane:ethyl acetate, and the combined filtrate and washes were concentrated to dryness at 37°C under reduced pressure. The residue was further dried under high vacuum for 1 h to give 24.4 g (76.1%) of crude compound of formula 1B' as a yellow oil. 1H NMR analysis indicated this material to be a 8.5:1 mixture of the compound of formula 1B' and its corresponding Z-isomer, the compound of formula 1*B'. This material was used directly in the next step without further purification.

In-process controls: NMR (CDCl₃) and TLC (3:1 dichloromethane:hexane; short-wave UV detection and PMA stain; R_{f} the compound of formula 12' = 0.55, R_{f} the compound of formula 1B' = 0.45 and R_{f} of the Z-isomer (compound of formula 1*B' = 0.35)

## Claims

1. A method of stereospecifically producing a compound of formula: 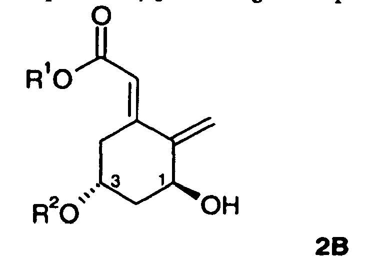
or its enantiomer 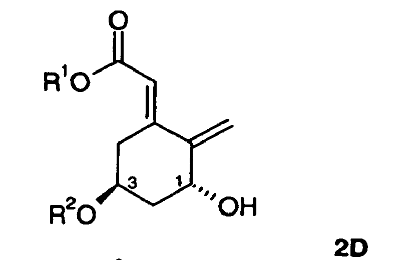
wherein R¹ is C₁-C₆ alkyl and R² is a hydroxy protecting group, which comprises reacting a compound of formula: 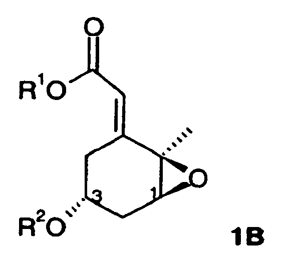
or its enantiomer, respectively, 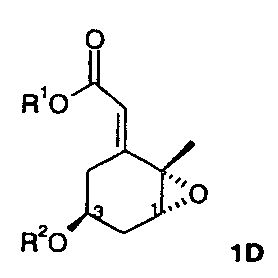
wherein
R¹ and R² are as above and
the stereochemistry of the compound of formula 1B, and the compound of formula 2B is the same at carbons 1 and 3, respectively, and the stereochemistry of both the compound of formula 1D and the compound of formula 2D is the same at carbons 1 and 3, respectively,
with a fluorinated alcohol having a pKₐ lower than about 9, in the presence of a palladium catalyst to yield the compound of formula 2B or 2D respectively.

2. The method of claim 1 , wherein the reacting is in the presence of a palladium catalyst that is palladium-phosphine catalyst.

3. The method of claim 2, wherein the reacting is in the presence of a palladium-phosphine catalyst that is a palladium-triarylphosphine.

4. The method of claim 3, wherein the reacting is in the presence of a palladium-triarylphosphine catalyst selected from the group consisting of palladium-triphenylphosphine, palladium-tris(2-methoxyphenyl)phosphine, palladium-tris(3-methoxyphenyl)phosphine, palladium-tris(4-methoxyphenyl)phosphine, palladium-tris(o-tolyl)phosphine, palladium-tris(m-tolyl)phosphine, palladium-tris(p-tolyl)phosphine, palladium-tris(4-fluorophenyl)phosphine, palladium-tris(p-trifluoromethylphenyl)-phosphine, and palladium-tris(2-furyl)phosphine.

5. The method of claim 1, wherein the reacting is in the presence of a palladium catalyst that is palladium-1,2-bis(diphenylphosphino) ethane.

6. The method of claim 1, wherein the reacting is with a fluorinated alcohol selected from the group consisting of: 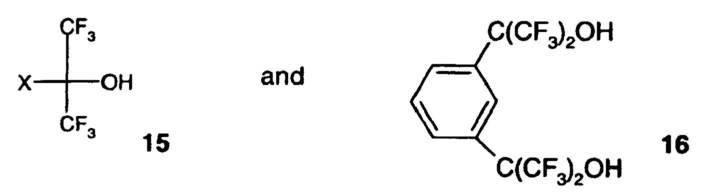
wherein X is phenyl or CF₃.

7. The method of claim 6, wherein the reacting is with a fluorinated alcohol which is:

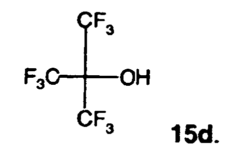

8. The method of claim 6, wherein the reacting is with a fluorinated alcohol which is:

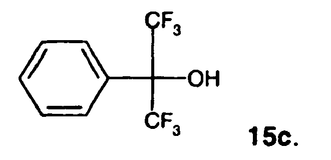

9. The method of claim 6, wherein the reacting is with a fluorinated alcohol which is:

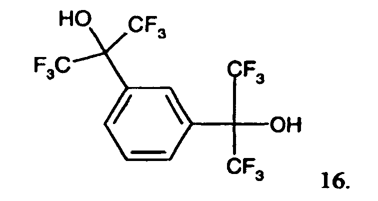

10. A compound having the structure: 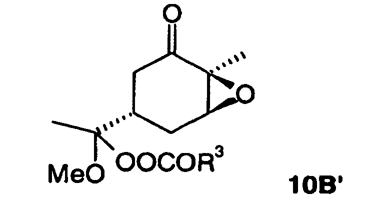
wherein R³ is C₁-C₆ alkyl, phenyl, 4-nitrophenyl, or CF₃; or its enantiomer.

11. A compound according to claim 10 having the structure: 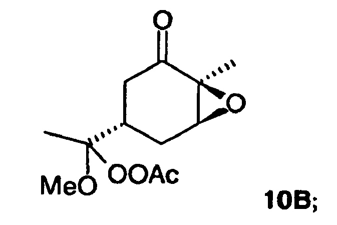
or its enantiomer.

12. A compound having the structure: 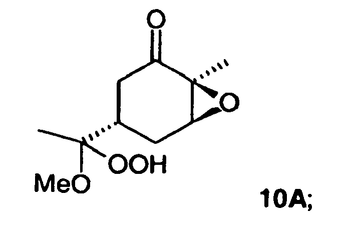
or its enantiomer.

13. A compound having the structure: 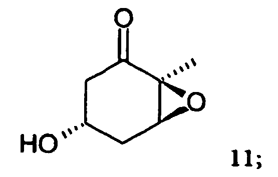
or its enantiomer.

14. A compound having the structure: 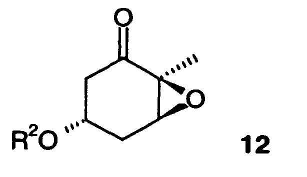
wherein
R² is a hydroxy protective group selected from the group consisting of trimethylsilyl, triethylsilyl, tripropylsilyl, triisopropylsilyl, *t*-butyldimethylsilyl, dimethylthexylsilyl, triphenylsilyl, and *t*-butyldiphenylsilyl; or its enantiomer.

15. A compound having the structure: 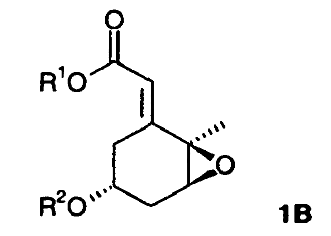
wherein
R¹ is C₁-C₆ alkyl and
R² is a hydroxy protective group selected from the group consisting of trimethylsilyl, triethylsilyl, tripropylsilyl, triisopropylsilyl, *t*-butyldimethylsilyl, dimethylthexylsilyl, triphenylsilyl, and *t*-butyldiphenylsilyl; or its enantiomer.

16. The compound according to claim 15 having the structure: 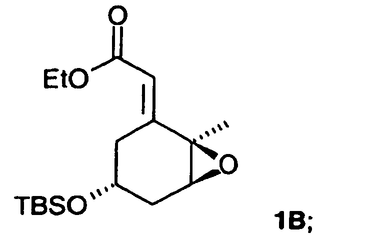
or its enantiomer.

## Patentansprüche

1. Verfahren zur stereospezifischen Herstellung einer Verbindung der Formel: 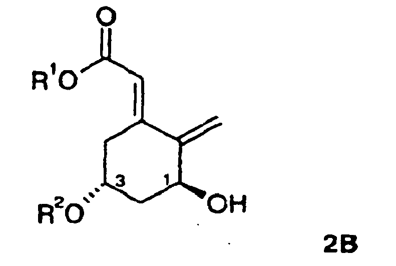
oder ihres Enantiomers 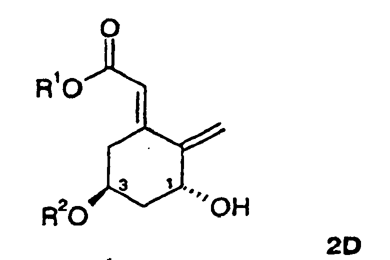
worin R¹ C₁-C₆-Alkyl ist und R² eine Hydroxyschutzgruppe ist,
umfassend das Umsetzen einer Verbindung der Formel: 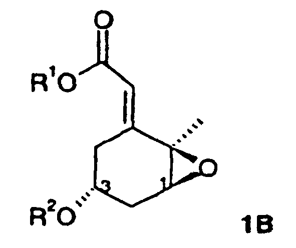
bzw. ihres Enantiomers 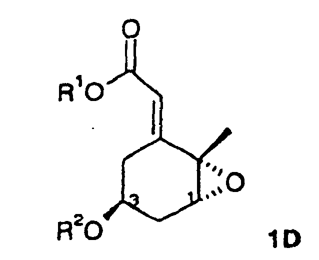
worin
R¹ und R² wie oben sind und
die Stereochemie der Verbindung der Formel 1B und der Verbindung der Formel 2B dieselbe an den Kohlenstoffen 1 bzw. 3 ist und die Stereochemie von sowohl der Verbindung der Formel 1D als auch der Verbindung der Formel 2D dieselbe an den Kohlenstoffen 1 bzw. 3 ist,
mit einem fluorierten Alkohol mit einem pKₐ-Wert von niedriger als etwa 9 in Gegenwart
eines Palladiumkatalysators, um die Verbindung der Formel 2B bzw. 2D zu erhalten.

2. Verfahren nach Anspruch 1, wobei die Umsetzung in Gegenwart eines Palladiumkatalysators, der ein Palladiumphosphinkatalysator ist, stattfindet.

3. Verfahren nach Anspruch 2, wobei die Umsetzung in Gegenwart eines Palladiumphosphinkatalysators, der ein Palladiumtriarylphosphin ist, stattfindet.

4. Verfahren nach Anspruch 3, wobei die Umsetzung in Gegenwart eines Palladiumtriarylphosphinkatalysators stattfindet, ausgewählt aus der Gruppe, bestehend aus Palladiumtriphenylphosphin, Palladium-tris(2-methoxyphenyl)phosphin, Palladium-tris(3-methoxyphenyl)phosphin, Palladium-tris(4-methoxyphenyl)phosphin, Palladium-tris(o-tolyl)phosphin, Palladium-tris(m-tolyl)phosphin, Palladium-tris(p-tolyl)phosphin, Palladium-tris(4-fluorphenyl)phosphin, Palladium-tris(p-trifluormethylphenyl)phosphin und Palladium-tris(2-furyl)phosphin.

5. Verfahren nach Anspruch 1, wobei die Umsetzung in Gegenwart eines Palladiumkatalysators, der Palladium-1,2-bis(diphenylphosphino)ethan ist, stattfindet.

6. Verfahren nach Anspruch 1, wobei die Umsetzung mit einem fluorierten Alkohol stattfindet, ausgewählt aus der Gruppe, bestehend aus: 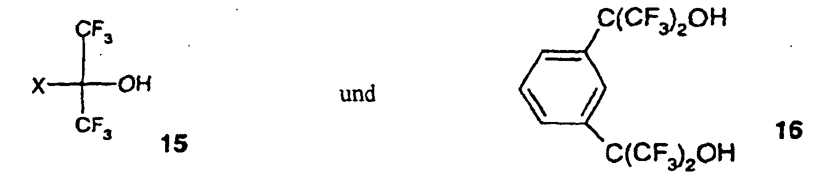
worin X Phenyl oder CF₃ ist.

7. Verfahren nach Anspruch 6, wobei die Umsetzung mit einem fluorierten Alkohol stattfindet, der: 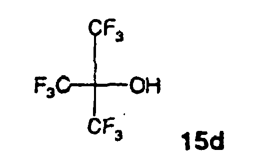
ist.

8. Verfahren nach Anspruch 6, wobei die Umsetzung mit einem fluorierten Alkohol stattfindet, der: 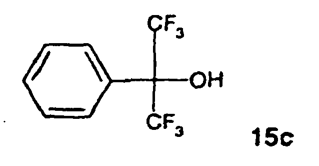
ist.

9. Verfahren nach Anspruch 6, wobei die Umsetzung mit einem fluorierten Alkohol stattfindet, der: 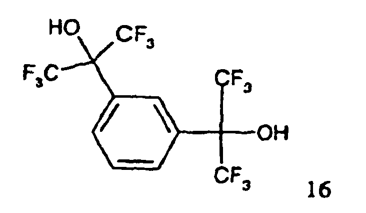
ist.

10. Verbindung mit der Struktur: 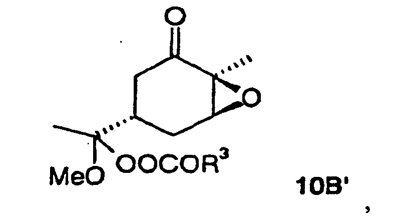
worin R³ C₁-C₆-Alkyl, Phenyl, 4-Nitrophenyl oder CF₃ ist; oder ihr Enantiomer.

11. Verbindung nach Anspruch 10 mit der Struktur: 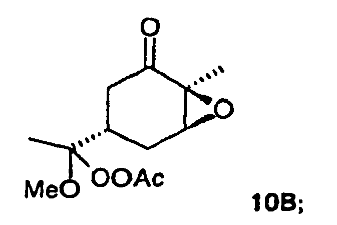
oder ihr Enantiomer.

12. Verbindung mit der Struktur: 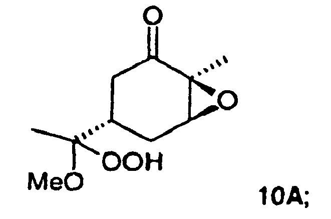
oder ihr Enantiomer.

13. Verbindung mit der Struktur: 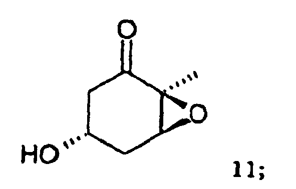
oder ihr Enantiomer.

14. Verbindung mit der Struktur: 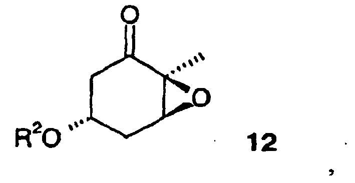
worin
R² eine Hydroxyschutzgruppe ist, ausgewählt aus der Gruppe, bestehend aus Trimethylsilyl, Triethylsilyl, Tripropylsilyl, Triisopropylsilyl, t-Butyldimethylsilyl, Dimethylhexylsilyl, Triphenylsilyl und t-Butyldiphenylsilyl; oder ihr Enantiomer.

15. Verbindung mit der Struktur: 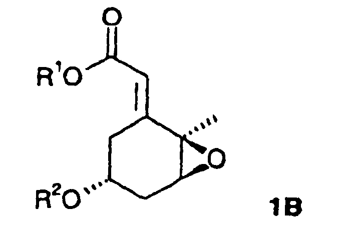
worin
R¹ C₁-C₆-Alkyl ist und
R² eine Hydroxyschutzgruppe ist, ausgewählt aus der Gruppe, bestehend aus Trimethylsilyl, Triethylsilyl, Tripropylsilyl, Triisopropylsilyl, t-Butyldimethylsilyl, Dimethylhexylsilyl, Triphenylsilyl und t-Butyldiphenylsilyl; oder ihr Enantiomer.

16. Verbindung nach Anspruch 15 mit der Struktur: 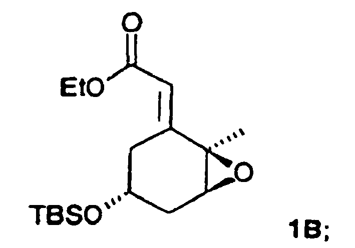
oder ihr Enantiomer.

## Revendications

1. Procédé de production stéréospécifique d'un composé de formule 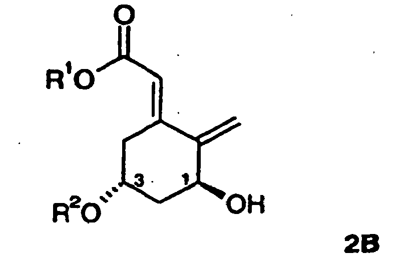
ou de son énantiomère 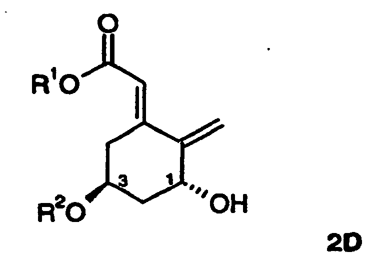
où R¹ est alkyle en C₁-C₆ et R² est un groupe protecteur d'hydroxy,
qui comprend la réaction d'un composé de formule 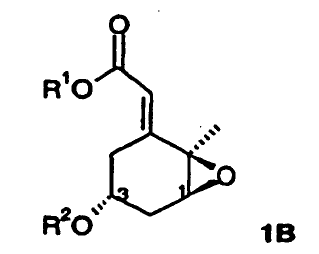
ou respectivement de son énantiomère 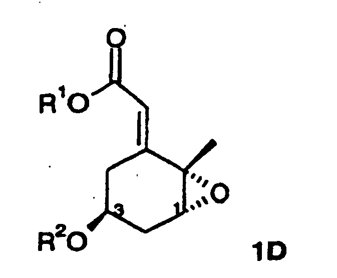
où R¹ et R² sont comme ci-dessus et
la stéréochimie du composé de formule **1B** et du composé de formule **2B** est la même au niveau des carbones 1 et 3, respectivement, et la stéréochimie du composé de formule **1D** et du composé de formule **2D** est la même au niveau des carbones 1 et 3, respectivement
avec un alcool fluoré ayant un pKₐ inférieur à environ 9, en présence d'un catalyseur à base de palladium, pour donner respectivement le composé de formule **2B** ou **2D.**

2. Procédé selon la revendication 1, dans lequel la réaction s'effectue en présence d'un catalyseur à base de palladium qui est un catalyseur palladium-phosphine.

3. Procédé selon la revendication 2, dans lequel la réaction s'effectue en présence d'un catalyseur palladium-phosphine qui est un palladium-triarylphosphine.

4. Procédé selon la revendication 3, dans lequel la réaction s'effectue en présence d'un catalyseur palladium-triarylphosphine choisi dans le groupe constitué par palladium-triphénylphosphine, palladium-tris(2-méthoxyphényl)phosphine, palladium-tris(3-méthoxyphényl)phosphine, palladium-tris(4-méthoxyphényl)-phosphine, palladium-tris(o-tolyl)phosphine, palladium-tris(m-tolyl)phosphine, palladium-tris(p-tolyl)phosphine, palladium-tris(4-fluorophényl)phosphine, palladium-tris(p-trifluorométhylphényl)phosphine, et palladium-tris(2-furyl)phosphine.

5. Procédé selon la revendication 1, dans lequel la réaction s'effectue en présence d'un catalyseur à base de palladium qui est du palladium-1,2-bis(diphénylphosphino)éthane.

6. Procédé selon la revendication 1, dans lequel la réaction s'effectue avec un alcool fluoré choisi dans le groupe constitué par 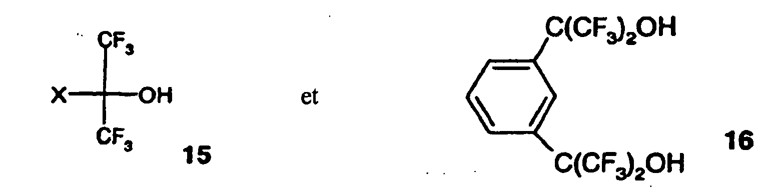
où X est phényle ou CF₃.

7. Procédé selon la revendication 6, dans lequel la réaction s'effectue avec un alcool fluoré qui est:

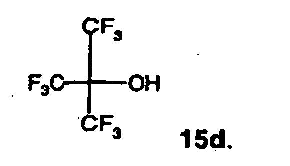

8. Procédé selon la revendication 6, dans lequel la réaction s'effectue avec un alcool fluoré qui est:

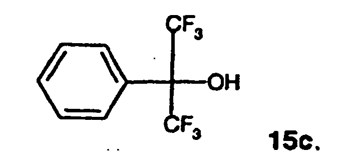

9. Procédé selon la revendication 6, dans lequel la réaction s'effectue avec un alcool fluoré qui est:

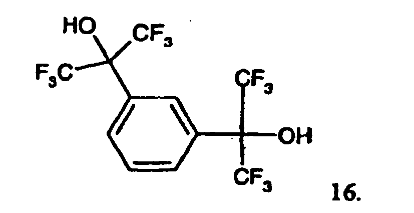

10. Composé ayant la structure: 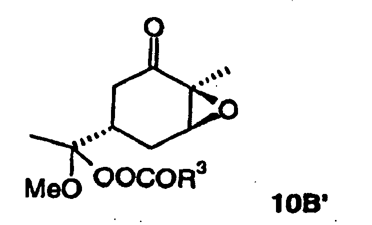
dans laquelle R³ est alkyle en C₁-C₆, phényle, 4-nitrophényle ou CF₃; ou son énantiomère.

11. Composé selon la revendication 10, ayant la structure: 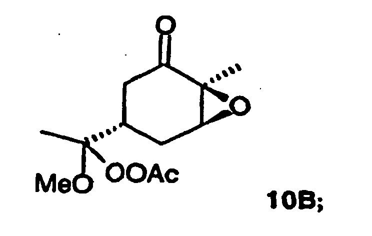
ou son énantiomère.

12. Composé ayant la structure: 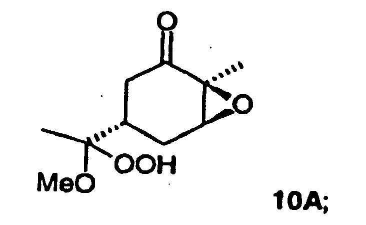
ou son énantiomère.

13. Composé ayant la structure: 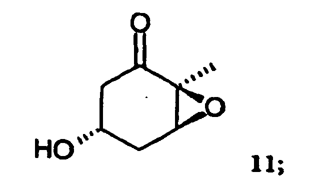
ou son énantiomère.

14. Composé ayant la structure: 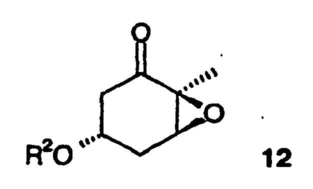
dans laquelle
R² est un groupe protecteur d'hydroxy choisi dans le groupe constitué par triméthylsilyle, triéthylsilyle, tripropylsilyle, triisopropylsilyle, t-butyldiméthylsilyle, diméthylthexylsilyle, triphénylsilyle et t-butyldiphénylsilyle;
ou son énantiomère.

15. Composé ayant la structure: 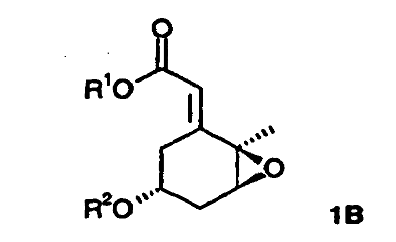
dans laquelle
R¹ est alkyle en C₁-C₆ et
R² est un groupe protecteur d'hydroxy choisi dans le groupe constitué par triméthylsilyle, triéthylsilyle, tripropylsilyle, triisopropylsilyle, t-butyldiméthylsilyle, diméthylthexylsilyle, triphénylsilyle et t-butyldiphénylsilyle;
ou son énantiomère.

16. Composé selon la revendication 15, ayant la structure: 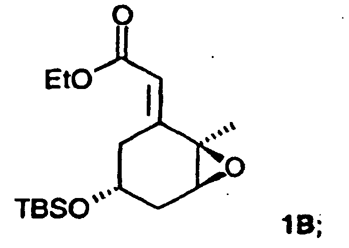
ou son énantiomère.
